**Europäisches Patentamt**

**European Patent Office**

**Office européen des brevets**

(19)

(11) Veröffentlichungsnummer : **0 159 462 B1**

(12)

# EUROPÄISCHE PATENTSCHRIFT

(45) Veröffentlichungstag der Patentschrift :
04.05.88

(51) Int. Cl.⁴ : **A 61 F   2/36**

(21) Anmeldenummer : **85100252.7**

(22) Anmeldetag : **12.01.85**

(54) Femurale Totalendoprothese für ein Hüftgelenk.

(30) Priorität : 23.03.84 DE 3410652
28.04.84 DE 3415934

(43) Veröffentlichungstag der Anmeldung :
30.10.85 Patentblatt 85/44

(45) Bekanntmachung des Hinweises auf die Patenterteilung : 04.05.88 Patentblatt 88/18

(84) Benannte Vertragsstaaten :
AT BE CH DE FR GB IT LI NL SE

(56) Entgegenhaltungen :
EP-A- 0 058 745
EP-A- 0 098 224
WO-A-83 /025 55
DE-A- 2 356 464
DE-A- 3 216 539
DE-A- 3 247 726
DE-C-   837 294
FR-A- 2 538 242
GB-A- 2 069 340
US-A- 2 719 522

(73) Patentinhaber : Orthoplant Endoprothetik GmbH
Leerkämpe 12
D-2800 Bremen 1 (DE)

(72) Erfinder : Schelhas, Klaus-Dieter
Semperstrasse 3
D-2000 Hamburg (DE)

(74) Vertreter : Eisenführ & Speiser
Martinistrasse 24
D-2800 Bremen 1 (DE)

EP 0 159 462 B1

**Beschreibung**

Die Erfindung betrifft eine femurale Totalendoprothese für ein Hüftgelenk, mit einem Femurkopf an einem Schaft, dessen Kern unterhalb des Femurkopfes einen senkrecht zur Medial-Lateral-Richtung abgeflachten, gekrümmten oberen Abschnitt, und einen sich konisch verjüngenden unteren Abschnitt aufweist, und mehrere in Längsrichtung des Schaftkerns verlaufende, sich zum distalen Schaftende hin verjüngende rippenförmige Ansätze trägt.

Aus der EP-A-0 098 224 ist eine derartige femurale Totalendoprothese bekannt, deren sich zum distalen Schaftende hin verjüngende rippenförmige Ansätze sich nach außen hin verbreitern, so daß zwischen den Ansätzen hinterschnittene Ausnehmungen oder Kanellierungen vorhanden sind, in die das nachwachsende Knochengewebe hineinwachsen soll, um einen festen Langzeitverbund zwischen der Prothese und dem Knochengewebe zu erzeugen. Die Ansätze verlaufen in Längsrichtung des Schaftkerns und verjüngen sich zum distalen Schaftende hin, um auch das einfache Entfernen einer eingewachsenen Prothese durch Aufbringung axialer Zugkräfte zu ermöglichen, falls eine Reoperation notwendig wird. Wegen der sich auswärts verbreiternden rippenförmigen Ansätze muß beim Einoperieren dieser bekannten Totalendoprothese ein Hohlraum im Knochen erzeugt werden, welcher der Außenkontur des Schafts der Prothese entspricht, wobei dann nach dem Einoperieren das Knochengewebe allmählich in die hinterschnittenen Nuten oder Kanellierungen hineinwächst. Der Verbund zwischen Prothese und Knochengewebe wird erst mit dem Nachwachsen des Knochengewebes fest, eine ausreichende Primärfixation nach der Operation wird nicht verwirklicht.

Aus der DE-A-3 216 539 ist eine femurale Totalendoprothese für ein Hüftgelenk bekannt, bei welcher der Schaftkern an seinem oberen Abschnitt mehrere große Rippen aufweist, die einen rechteckförmigen Querschnitt und einen relativ großen Abstand voneinander besitzen. In den Zwischenräumen zwischen den Rippen bleibt beim Einoperieren der Prothese dank entsprechender Formung des Knochenhohlraums Knochengewebe stehen, welches eine gewisse Primärfixation der Prothese gegenüber Rotationskräften bewirkt. Um ein Herausoperieren der eingewachsenen Prothese ohne größere Beschädigung des Knochengewebes zu ermöglichen, verlaufen die Rippen parallel zueinander längs des Schaftkerns. Die verwirklichte Primärfixation gegenüber Rotationskräften hat zur Folge, daß der Schaftkern wegen der relativ großen Rippen dünn ist und sich daher nicht im tragenden Knochengewebe abstützen kann, so daß die Stützkräfte im wesentlichen über die Rippen übertragen werden müssen. Die Krafteinleitung zwischen dem Knochengewebe und der Prothese erfolgt bei Kräften mit Radialkomponenten nur an der Außenkante der Rippen, wo erhebliche Flächenpressungen entstehen, welche den Verbund zwischen Prothese und Knochengewebe beeinträchtigen.

Aus der EP-A-0 058 745 ist eine femurale Totalendoprothese mit einem blattartigen Schaft bekannt, der im Knochen einzementiert wird. Um die Haftfähigkeit des Schaftes im Knochenzementbett zu erhöhen, sind die Blattseiten mit Rillen versehen, die etwa parallel zur Längsmittelachse und zum konischen Teil der Oberflächen der Blattseiten verlaufen, und deren Höhe zum distalen Schaftende hin abnimmt.

Aufgabe der vorliegenden Erfindung ist es, eine zementfrei zu implantierende femurale Totalendoprothese der eingangs genannten Art derart weiterzubilden, daß sowohl eine gute Primärfixation als auch Langzeitfixation der Prothese im Knochengewebe erzielt wird.

Diese Aufgabe wird bei der femuralen Totalendoprothese der eingangs genannten Art erfindungsgemäß dadurch gelöst, daß die rippenförmigen Ansätze einen dreieckförmigen Querschnitt besitzen, nur im ventralen und im dorsalen Bereich des Schaftkerns angeordnet und so geformt sind, daß ihre Scheitel einen konstanten Abstand voneinander aufweisen.

Die Vorteile der Erfindung liegen insbesondere darin, daß die rippenförmigen Ansätze, deren Scheitel einen konstanten Abstand voneinander aufweisen, aufgrund ihrer Formgebung beim Implantieren in die harte Spongiosa eindringen oder gar einschneiden, und dieses Knochengewebe — wegen der sich zum proximalen Schaftende hin verengenden Zwischenräumen zwischen den Ansätzen — beim allmählichen Einführen der Totalendoprothese zunehmend verdichten. Aufgrund dieser Formgebung braucht der die Prothese aufnehmende Hohlraum im Knochen nur auf das Kernmaß des Schaftes ausgefräst zu werden, und die Verdichtung des Knochengewebes in den Zwischenräumen zwischen den Ansätzen erzeugt eine hohe Primärfixation insbesondere gegen Rotationsbeanspruchung unmittelbar nach der Operation. Da die rippenförmigen Ansätze außerdem nur im ventralen und im dorsalen Bereich des Schaftkerns angeformt sind, geht der Schaftkernquerschnitt, der am proximalen Schaftende eine ovale Form mit mediallateral ausgerichteter Längsachse besitzt, zum distalen Schaftende hin in eine ovale Form über, deren Längsachse ventral-dorsal ausgerichtet ist. Dadurch wird die Primärfixation des Schaftes zusätzlich erhöht. Durch die hinterschneidungsfreie Form der Ansätze läßt sich die Totalendoprothese außerdem problemlos — ohne Zerstörung von Knochengewebe — aus dem Markkanal herausziehen, wenn dies notwendig ist.

Bevorzugt nimmt die Breite, und besonders bevorzugt auch die Höhe der rippenförmigen Ansätze zum distalen Schaftende hin auf etwa die Hälfte bis ein Drittel des am proximalen Schaftende vorhandenen Wertes ab, und der Abstand zwischen den Scheiteln benachbarter Ansätze liegt bevorzugt zwischen 3 mm und 6 mm. Bei dieser Dimensionierung der Ansätze wird eine besonders feste Verankerung der Prothese dann erreicht, wenn die zu implantierende Prothese so ausgewählt wird, daß ihr Kraftkern in die tragende, härtere Spongiosa hineinragt.

Die rippenförmigen Ansätze erstrecken sich in einer bevorzugten Ausführungsform der Erfindung über die jeweils gesamte verfügbare Länge des Schaftkernes. Die im Bereich des Adam'schen Bogens vorhandenen Ansätze sind — aufgrund der Krümmung des Schaftkernes entsprechend kurz, einige Ansätze erstrecken sich dagegen bis zum im wesentlichen kreiskonzentrisch auslaufenden distalen Schaftende. Alternativ können die rippenförmigen Ansätze in vorgegebenem Abstand vom distalen Schaftende enden.

Bevorzugt ist an der lateralen Stirnseite des proximalen Schaftendes ein sich medial-lateral ausstreckender nasenförmiger Ansatz angeformt, der an seinem dem Schaft abgekehrten lateralen Endabschnitt einen querverlaufenden Flansch trägt, der bevorzugt beiderseits des nasenförmigen Ansatzes vorsteht und symmetrisch zu diesem ausgebildet ist. Es hat sich dabei als zweckmäßig erwiesen, daß dieser Flansch — in Anpassung an die Form des mehr oder weniger parallel zum Flansch verlaufenden proximalen Schaftendes — nach unten hin verjüngt. Der querverlaufende, gekrümmte Flansch setzt die laterale Stirnseite des Schaftkerns nach oben hin fort und kommt bei der Implantation der Prothese sogleich mit dem Knochengewebe in formschlüssigen Eingriff, wodurch sich eine zusätzliche Verankerung gegen Rotationsbeanspruchung und eine zusätzliche Stützverankerung am Knochengewebe ergibt.

Bevorzugt werden in dem nasenförmigen Ansatz und/oder dem querverlaufenden Flansch Durchgangsöffnungen eingearbeitet, in die das Knochengewebe nach dem Implantieren allmählich hineinwächst, wodurch im lateralen Bereich des proximalen Schaftendes ein besonders fester Verbund zwischen Prothese und Knochen entsteht.

Vorteilhafte Weiterbildungen der Erfindung sind durch die Merkmale der abhängigen Ansprüche gekennzeichnet.

Im folgenden wird ein Ausführungsbeispiel der Erfindung anhand der Zeichnungen näher erläutert. Es zeigen :

Fig. 1 eine Seitenansicht einer erfindungsgemäßen Hüftgelenk-Totalendoprothese ohne Femurkopf ;

Fig. 2 eine Ansicht der Prothese gemäß Fig. 1 in Richtung des Pfeiles II in Fig. 1 gesehen ;

Fig. 3 eine Teilansicht der Prothese gemäß den Fig. 1 und 2 in Richtung des Pfeiles III in Fig. 1 gesehen ;

Fig. 4 einen Querschnitt durch den Schaft der Prothese gemäß den Fig. 1 bis 3 in Richtung der Schnittlinie IV-IV gesehen ;

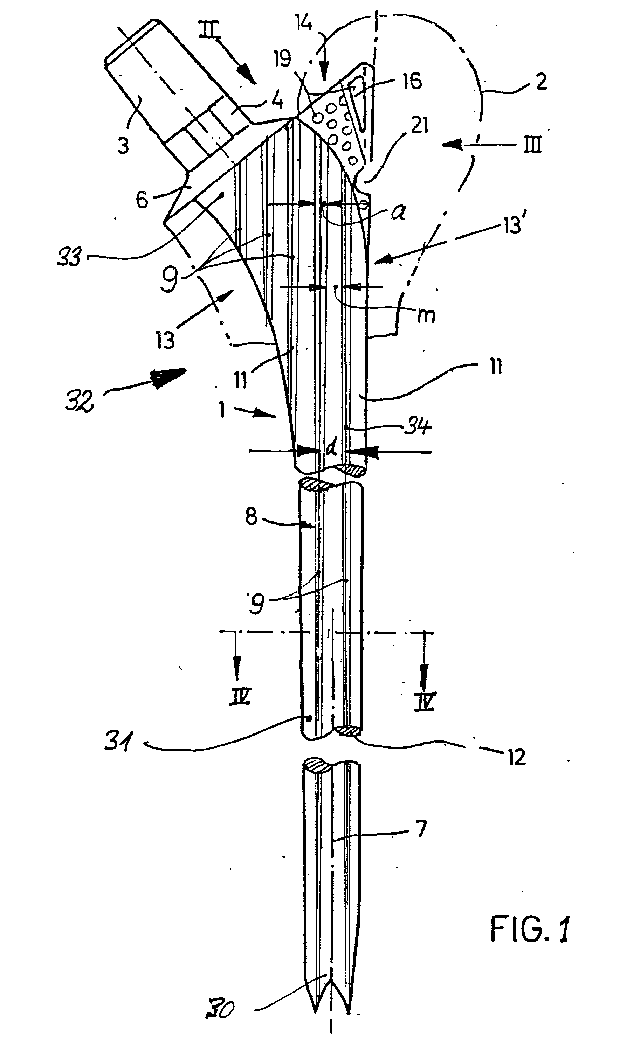

Die Fig. 1 bis 4 zeigen eine Totalendoprothese für ein Hüftgelenk mit einem im Markkanal des Femurknochens zu verankernden Schaft 1. Der Femurknochen ist im wesentlichen nicht dargestellt und lediglich in seinem oberen Endabschnitt — nach Resektion des natürlichen Femurkopfes unter einem Winkel von 50° — mit einer strichpunktierten Linie 2 angedeutet.

Der Schaft 1 ist an seinem oberen Endabschnitt mit einem kugelförmigen Femurkopf versehen, der in der Zeichnung nicht dargestellt ist. Der nicht dargestellte Femurkopf wird auf einen konischen Zapfen 3 aufgesteckt, um auf diese Weise unterschiedliche Kugelkopfgrößen verwirklichen zu können. Unterhalb des Zapfens 3 befindet sich ein integral mit diesem ausgebildeter Mehrkant 4, an dem die Prothese mit einem entsprechenden Schlüssel gehalten bzw. gedreht werden kann, und unterhalb des Mehrkant 4 befindet sich ein flanschförmiger Kragen 6.

Der unterhalb des Femurkopfes bzw. des Kragens 6 liegende obere Abschnitt 32 des Schaftkerns 12 ist in einer sich senkrecht zur Medial-Lateral-Richtung liegenden Richtung abgeflacht und in seinem oberen Abschnitt in Medialrichtung einwärts gekrümmt, wobei dieser gekrümmte Abschnitt sich verjüngend in den unteren Schaftabschnitt 31 übergeht, der sich von oben nach unten weiterhin konisch verjüngt und einen im wesentlichen kreisförmigen Querschnitt aufweist und im distaler Schaftende 30 endet.

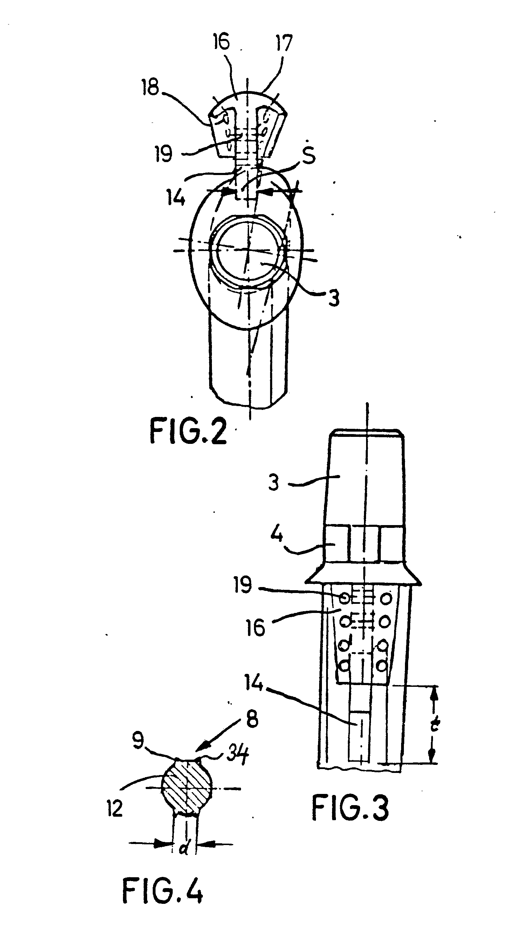

Die Oberfläche des Schaftes 1 ist zu einem großen Teil mit einer sich im wesentlichen in Längsrichtung gemäß der Achse 7 des unteren Abschnittes erstreckenden Profilierung 8 versehen, die aus rippenförmigen Ansätzen 9 besteht, welche sich von der glatten Basisumfangsfläche 11 des Schaftkernes 12 aus nach außen erstrecken und integral mit dem Schaftkern 12 ausgebildet sind. In der Ausgestaltung gemäß Fig. 4 sind die rippenförmigen Ansätze 9 dreiecksförmig ausgebildet mit leicht abgerundeten Scheiteln.

Die rippenförmigen Ansätze 9 verjüngen sich vom proximale Schaftende 33 zum unterem, distalen Schaftende 30 hin, indem sowoh die Höhe h als auch die Breite a zum distalen Schaftende 30 hin abnehmen. Die Breite a, die an dem Fuß der Ansätze 9, unmittelbar am Schaftkern 12 gemessen wird, und die Höhe h reduzieren sich dabei nach unten hin bis auf etwa die Hälfte oder ein Drittel ihres Anfangswertes. So nimmt zum Beispiel die Breite a von etwa 3,5 auf 1,0 mm, und die Höhe h von zum Beispiel 1,5 auf 0,5 mm ab.

Die rippenförmigen Ansätze 9 besitzen — von Fuß zu Fuß — einen kleinsten gegenseitigen Abstand m von zum Beispiel 2,5 mm, der aufgrund der abnehmenden Breite der rippenförmigen Ansätze zum distalen Schaftende 30 hin zunimmt, während die Scheitel 34 der Ansätze 9 über die gesamte Länge der Ansätze 9 einen konstanten Abstand 9 voneinander besitzen und — bis auf die geringe Konizität der Schaftkernes — parallel Längsachse 7 des Schaftkernes 12 verlaufen.

Diese Ausgestaltung der rippenförmigen Ansätze 9 führt beim Implantieren zu einer Art Verkeilung der rippenförmigen Ansätze 9 im natürlichen Knochengewebe, derart, daß auch das Knochengewebe zwischen den Ansätzen 9 beim Einsetzen der Prothese zunehmend verdichtet und damit verfestigt wird. Zum anderen läßt sich bei einer erforderlichen Reoperation eine solche Prothese trotz der mit ihr im implantierten Zustand erreichten optimalen Rotationssicherung leicht wieder herausziehen, wenn die Prothese erst einmal in Längsrichtung um eine verhältnismäßig geringfügige axiale Länge gelockert worden ist. Außerdem schneiden sich die Ansätze 9 im Knochengewebe ihre Sitzkanäle beim Einsetzen der Prothese selbst und belasten dabei das Knochengewebe nur ganz allmählich zunehmend stärker.

Die Länge der rippenförmigen Ansätze 9 erstreckt sich vom proximalen Schaftende 32 über die gesamte Länge des Schaftkernes 12. An den beiden einander gegenüberliegenden Stirnseiten 13 und 13′ des Schaftes 1 sind keine rippenförmigen Ansätze 9 vorhanden.

An der lateralen Stirnseite des oberen Schaftabschnittes 32 befindet sich ein nasenförmiger Ansatz 14, dessen Dicke s kleiner ist als die Dicke des oberen Schaftabschnittes 32. Der nasenförmige Ansatz 14 weist an seinem dem Schaft 1 abgekehrten lateralen Ende einen quer verlaufenden Flansch 16 auf, der symmetrisch zu dem nasenförmigen Ansatz 14 angeordnet ist, sich also beiderseits des Ansatzes 14 über diesen hinaus erstreckt. Der Flansch 16 verjüngt sich von oben nach unten, wie aus Fig. 3 erkennbar ist, und ist sowohl an seiner Außenseite 17 als auch an seiner Innenseite 18 zum Schaft 1 hin gekrümmt ausgebildet. Der Flansch 16 ist ebenso wie der nasenförmige Ansatz 14 mit Durchgangsöffnungen 19 versehen. Der Flansch 16 endet mit einem Abstand t zum unteren Ende des nasenförmigen Ansatzes 14. Letzterer ist unterhalb des Flansches 16 mit einer halbkreisförmigen Ausnehmung 21 versehen.

Implantation der Prothese wird zunächst der natürliche Femurkopf unter einem Winkel von 50° resiziert und der Trochanter mit einer nutförmigen Ausnehmung versehen, so daß die Prothese von oben her in den Femurknochen 2 eingeführt werden kann, wobei die Prothese beim Einführen mittels eines entsprechenden Schlüssels am Mehrkant 4 um ihre Achse 7 gedreht werden kann. Dabei kommen die rippenförmigen Ansätze 9 ebenso wie der aus dem nasenförmigen Ansatz 14 und dessen Flansch 16 bestehende T-förmige « Flügel » sogleich bei der Implantation mit dem Knochengewebe in formschlüssigen Eingriff, so daß sich unverzüglich selbst bei der zementfreien Implantation eine hohe Drehsicherheit ergibt.

Selbst wenn im Verlaufe der Zeit Gewebe in die Nuten der Profilierung 8 sowie die Durchgangsöffnungen 19 einwächst, ist erforderlichenfalls eine Entfernung des Schaftes aus dem Knochengewebe in Richtung der Achse 7 ohne beachtliche Beschädigung des Gewebes möglich, da sich — bis auf ein Einwachsen in den Durchgangsöffnungen 19 — an der Profilierung 8 keine quer oder schräg zur Längsachse 7 verlaufenden Hinterschneidungen befinden.

Der T-förmige Abschnitt 14, 16 bezieht das Trochantermassiv festigkeitsmäßig praktisch voll ein und sorgt demgemäß für eine entsprechende Entlastung des schaftes 1 bei dessen Belastung, da die gegenüber dem Adam'schen Bogen bei Belastung stets herrschenden Zugkräfte z. T. von dem natürlichen Gewebe selbst übertragen werden können.

Der Flansch 16 des nasenförmigen Ansatzes 14 ist mit zwei symmetrisch angeordneten, fensterförmigen Durchgangsöffnungen 19 versehen.

**Patentansprüche**

1. Femurale Totalendoprothese für ein Hüftgelenk, mit einem Femurkopf an einem Schaft, dessen Kern unterhalb des Femurkopfes einen senkrecht zur Medial-Lateral-Richtung abgeflachten, gekrümmten oberen Abschnitt, und einen sich konisch verjüngenden unteren Abschnitt aufweist, und mehrere in Längsrichtung des Schaftkerns verlaufende, sich zum distalen Schaftende hin verjüngende rippenförmige Ansätze trägt, dadurch gekennzeichnet, daß die rippenförmigen Ansätze (9) einen dreieckförmigen Querschnitt besitzen, nur im ventralen und im dorsalen Bereich des Schaftkerns (12) angeordnet und so geformt sind, daß ihre Scheitel (34) einen konstanten Abstand (d) voneinander aufweisen.

2. Prothese nach Anspruch 1, dadurch gekennzeichnet, daß die Breite (a) der rippenförmigen Ansätze (9) zum distalen Schaftende (30) hin auf etwa ein Drittel ihres am proximalen Schaftende (33) vorhandenen Wertes abnimmt.

3. Prothese nach Anspruch 1 oder 2, dadurch gekennzeichnet, daß die Höhe der rippenförmigen Ansätze (9) zum distalen Schaftende (30) hin auf etwa die Hälfte bis ein Drittel der am proximalen Schaftende (33) vorhandenen größten Höhe abnimmt.

4. Prothese nach einem der vorstehenden Ansprüche, dadurch gekennzeichnet, daß die rippenförmigen Ansätze (9) an ihrem Scheitel (34) abgerundet sind.

5. Prothese nach einem der vorstehenden Ansprüche, dadurch gekennzeichnet, daß der konstante Abstand (d) zwischem dem Scheitel (34) benachbarter rippenförmiger Ansätze (9) etwa zwischen 3 und 6 mm beträgt.

6. Prothese nach einem der vorstehenden Ansprüche, dadurch gekennzeichnet, daß die rippenförmigen Ansätze (9) mit Abstand zum distalen Schaftende (30) enden.

7. Prothese nach einem der Ansprüche 1 bis 5, dadurch gekennzeichnet, daß die rippenförmigen Ansätze (9) sich über die jeweils gesamte verfügbare Länge des Schaftkerns (12) erstrecken.

8. Prothese nach einem der vorstehenden Ansprüche, dadurch gekennzeichnet, daß an der lateralen Stirnseite des proximalen Schaftendes (33) ein sich medial/lateral erstreckender nasenförmiger Ansatz (14) angeordnet ist, der an seinem dem Schaft (1) abgekehrten lateralen Endabschnitt einen querverlaufenden Flansch (16) trägt.

9. Prothese nach Anspruch 8, dadurch gekennzeichnet, daß sich der Flansch (16) nach unten hin verjüngt.

10. Prothese nach Anspruch 8 oder 9, dadurch gekennzeichnet, daß der Flansch (16) wenigstens an seiner Außenseite (17) zum Schaft (1) hin gekrümmt ausgebildet ist.

11. Prothese nach einem der Ansprüche 8 bis 10, dadurch gekennzeichnet, daß der nasenförmige Ansatz (14) und/oder der Flansch (16) mit Durchgangsöffnungen (19) versehen ist.


**Claims**

1. Complete femoral endoprosthesis for a hip joint, having a femur head on a stem, the core of, which, below the femur head, comprises a curved upper section flattened perpendicularly of the medial-lateral direction, and a conically tapering lower section, and carries a plurality of rib-like projections which extend longitudinally of the stem core and taper towards the distal end of the stem, characterised in that the rib-like projections (9) have a triangular cross-section, are arranged only in the ventral region and in the dorsal region of the stem core (12) and are so formed that their apices (34) have a constant spacing (d) from one another.

2. Prosthesis according to claim 1, characterised in that the width (a) of the rib-like projections (9) decreases towards the distal end (30) of the stem to about one third of their value existing at the proximal end (33) of the stem.

3. Prosthesis according to claim 1 or 2, characterised in that the height of the rib-like projections (9) decreases towards the distal end (30) of the stem to about a half to a third of the largest height existing at the proximal end (33) of the stem.

4. Prosthesis according to one of the preceding claims, characterised in that the rib-like projections (9) are rounded at their apex or tip (34).

5. Prosthesis according to one of the preceding claims, characterised in that the constant spacing (d) between the tip (34) of adjacent rib-like projections (9) is approximately between 3 and 6 mm.

6. Prosthesis according to one of the preceding claims, characterised in that the rib-like projections (9) terminate at a distance from the distal end (30) of the stem.

7. Prosthesis according to one of claims 1 to 5, characterised in that the rib-like projections (9) extend over the respective total available length of the stem core (12).

8. Prosthesis according to one of the preceding claims, characterised in that the lateral face of the proximal end (33) of the stem has arranged thereon a medially-laterally extending nose-like projection (14), which carries a transversely extending flange (16) on its lateral end section facing away from the stem (1).

9. Prosthesis according to claim 8, characterised in that the flange (16) is tapered downwardly.

10. Prosthesis according to claim 8 or 9, characterised in that the flange (16), at least on its outer side (17), is made curved towards the stem (1).

11. Prosthesis according to one of the claims 8 to 10, characterised in that the nose-like projection (14) and/or the flange (16) is formed with passage openings (19).


**Revendications**

1. Endoprothèse fémorale totale pour une articulation de la hanche, munie d'une tête de fémur sur une tige dont l'âme présente, sous la tête du fémur, une zone supérieure courbée, aplatie perpendiculairement à la direction médiale latérale et une zone inférieure se rétrécissant suivant une forme cônique, l'âme de la tige portant plusieurs saillies en forme de nervures s'étendant dans le sens longitudinal de l'âme de la tige et dont la hauteur diminue vers l'extrémité distale de la tige, caractérisée en ce que les saillies (9) en forme de nervures comportant une section de forme triangulaire, ne sont disposées que dans la partie ventrale et la partie dorsale de l'âme de la tige et sont conformées de telle sorte que leurs sommets (34) présentent entre eux un écart constant (d).

2. Prothèse selon la revendication 1, caractérisé en ce que la largeur (a) des saillies en forme de nervures décroît sur l'extrémité distale de la tige (30) jusqu'à environ un tiers de sa valeur à l'extrémité proximale de la tige (33).

3. Prothèse selon la revendication 1 ou 2, caractérisée en ce que la hauteur des saillies en forme de nervures (9) décrit vers l'extrémité distale de la tige (30) jusqu'à une valeur allant de la moitié au tiers de la plus grande hauteur existant à l'extrémité proximale de la tige (33).

4. Prothèse selon une des revendications précédentes, caractérisée en ce que les saillies en forme de nervures (9) sont arrondies à leur sommet (34).

5. Prothèse selon une des revendications précédentes, caractérisée en ce que l'écart (d) constant entre les sommets (34) de saillies (9) en forme de nervures voisines se situe entre 3 et 6 mm environ.

6. Prothèse selon une des revendications précédentes, caractérisée en ce que les saillies (9) en forme de nervures prennent fin à une certaine distance de l'extrémité distale de la tige (30).

7. Prothèse selon une des revendications 1 à 5, caractérisée en ce que les saillies (9) en forme de nervures s'étendent sur toute la longueur disponible de l'âme de la tige (12).

8. Prothèse selon une des revendications précédentes, caractérisée en ce qu'on dispose sur la face frontale latérale de l'extrémité proximale de la tige (33), une saillie (14) en forme de talon s'étendant suivant une direction médiale/latérale, qui supporte une bride (16) orientée transversalement dans sa zone d'extrémité latérale opposée à la tige (1).

9. Prothèse selon la revendication 8, caractérisée en ce que la bride (16) se rétrécit vers le bas.

10. Prothèse selon la revendication 8 ou 9, caractérisée en ce que la bride (16) est réalisée avec une courbure, au moins sur sa face extérieure par rapport à la tige.

11. Prothèse selon une des revendications 8 à 10, caractérisée en ce que la saillie (14) en forme de talon et/ou la bride (16) est pourvue d'ouvertures de passage (19).

FIG.1

**FIG.2**

**FIG.3**

**FIG.4**